# EUROPEAN PATENT SPECIFICATION

(11) **EP 3 209 199 B1**
(45) Date of publication and mention of the grant of the patent: **24.04.2019**
(21) Application number: 15794384.6
(22) Date of filing: 23.10.2015
(51) Int. Cl.: A61B 5/0215, A61B 5/026, A61M 25/00, A61M 25/09

(54) **MICROCATHETER SENSOR DESIGN FOR MINIMIZING PROFILE AND IMPACT OF WIRE STRAIN ON SENSOR**
ENTWURF EINES MIKROKATHETERSENSORS ZUR MINIMIERUNG DES PROFILS UND EINFLUSSES DES DRAHTZUGES AUF DEN SENSOR
CONCEPTION DE CAPTEUR DE MICROCATHÉTER POUR RÉDUIRE AU MINIMUM LE PROFIL ET L'IMPACT D'UNE TENSION DE FIL SUR LE CAPTEUR

(30) Priority: 24.10.2014 US 201462068052 P
(43) Date of publication of application: 30.08.2017
(73) Proprietor: Medtronic Vascular Inc., Santa Rosa, CA 95403 (US)
(72) Inventor: MURPHY, Christopher, Santa Rosa, California 9540 (US); MCCAFFREY, Gerry, Santa Rosa, California 95403 (US); SWEENEY, Fiachra, Santa Rosa, California 95403 (US); WARD, Sean, Santa Rosa, California 95403 (US)
(74) Representative: Zimmermann & Partner Patentanwälte mbB
(86) International application number: PCT/US2015/057058
(87) International publication number: WO 2016/065227

(56) References cited:
- EP-A1- 1 658 808
- WO-A1-2014/025255
- NL-C- 2 009 285
- US-A- 5 050 297
- US-A1- 2014 187 984
- US-B1- 6 733 459

## Description

### FIELD OF THE INVENTION

The invention relates to systems for determining a pressure gradient across a lesion of a vessel for calculating a Fractional Flow Reserve.

### BACKGROUND OF THE INVENTION

The severity of a stenosis or lesion in a blood vessel may be assessed by obtaining proximal and distal pressure measurements relative to the given stenosis and using those measurements for calculating a value of the Fractional Flow Reserve (FFR). FFR is defined as the ratio of a first pressure measurement (P_{d}) taken on the distal side of the lesion and to a second pressure measurement taken on the proximal side of the lesion usually within the aorta (Pₐ). Conventionally, a sensor is placed on the distal portion of a guidewire or FFR wire to obtain the first pressure measurement P_{d}, while an external pressure transducer is fluidly connected via tubing to a guide catheter for obtaining the second or aortic (AO) pressure measurement Pₐ. Calculation of the FFR value provides a lesion specific index of the functional severity of the stenosis in order to determine whether the blockage limits blood flow within the vessel to an extent that treatment is needed. An optimal or normal value of FFR in a healthy vessel is 1.00, while values less than about 0.80 are generally deemed significant and in need of an interventional treatment. Common interventional treatment options include balloon angioplasty and/or stent implantation.

If an interventional treatment is required, the interventional device, such as a balloon catheter, is tracked over a guide wire to the site of the lesion. Conventional FFR wires generally are not desired by clinicians to be used as guide wires for such interventional devices. Accordingly, if an intervention treatment is required, the clinician generally removes the FFR wire, inserts a conventional guide wire, and tracks the interventional device to the treatment site over the conventional guide wire.

The mounting of a pressure sensor on the distal end of a catheter, such as a microcatheter makes it difficult to isolate the pressure sensor from bending stresses experienced as a result of interaction between the pressure sensor and the housing of the catheter. Due to the high sensitivity and size of the pressure sensor used in this application, any stress placed on the pressure sensor can cause a distortion of the sensor resulting in an incorrect pressure reading or bend error. Accordingly, there remains a need for a microcatheter to obtain pressure measurements suitable for use in calculating an FFR value for a given stenosis, whereby the clinician may use a conventional or preferential guidewire instead of a FFR guidewire. In addition, there remains a need for a FFR microcatheter to minimize both the profile of the catheter and the bending stresses experienced by the pressure sensor.

US Patent US 6733459 B1 describes a balloon catheter. The balloon catheter for intra-aortic balloon pump which measures accurate blood pressure at the predetermined position of balloon introduced percutaneously and ensures the second lumen when catheter is in use. The balloon catheter comprised of a Y-shaped connector having gas supply port and an electric circuit, a hollow catheter tube one of which end is connected to the Y-shaped connector, a balloon one of which end is connected to the other end of the catheter tube, a hollow central tube one end of which is connected to one end of the balloon and extended through inside of the catheter tube and the balloon. The balloon catheter includes a pressure detecting element which enables to measure the pressure on the surface of a top end chip connecting the balloon and the central tube. The balloon catheter provided with a detachable connecting device transmitting output signal from the pressure detecting element to an external control device.

The publication WO 2014025255 A1 discloses a pressure sensor catheter and a method for measuring pressure on a distal side of a flow constriction, such as a stenosis, in a body lumen or measuring a pressure difference over the flow constriction. The catheter comprises a pressure transfer tube for passing through the constriction, the tube being connected to and extending from a pressure sensor for transferring the pressure at the distal side of the constriction via the tube to the sensor, and a catheter body for introduction of the catheter into the body lumen, wherein the catheter body comprises the sensor at a sensor position, such that the sensor in use is positioned in the body lumen, wherein a distal end region of the catheter comprising the distal end region of the pressure transfer tube has a smaller maximum outer diameter than the outer diameter of the catheter at the sensor position.

US Patent Application US 5050297 (A) discloses A catheter sensor assembly comprising: a) an elongate member with a distal end to ease insertion of the catheter sensor assembly and a proximal extension shaped for fitment of the member into a lumen of a catheter tube, the distal end and the proximal extension joined by an intermediate part; b) a lower section having a curved bottom defining the tubular shape of the elongate member so that when the proximal extension is fit within the lumen the tubular member extends distally therefrom; c) a passage opened in a first direction and extending along the proximal extension and across the intermediate part; d) a sensor carried upon and overlying the passage intermediate part near the distal end, the sensor having a bottom surface of substantially planar configuration and a top with an open recess so that the top recess faces in the first direction when the sensor covers the passage with the bottom planar surface in communication with a distal portion of the passage and the lumen; e) conductors attached to an area of the sensor bottom planar surface near the proximal extension for transmitting signals from the sensor through the lumen; and f) an adhesive sealant between the sensor and the intermediate part about the bottom planar surface for securing the sensor to the member on the intermediate part near the distal end with the sealant also across the conductors and the area of the sensor that the sealant does not close the entire passage.

US Patent Application US 20140187984 A1 discloses a guidewire system for treating a patient including a sensor assembly for detecting a physiological characteristic of a patient and a hypotube sized for insertion into vasculature of the patient and having an integrated sensor mount formed therein for predictably locating the sensor during assembly. The hypotube may also have a wall structure and a lumen, and the sensor mount may be formed within the wall structure of the hypotube and may include a first mechanical stop configured to limit movement of the sensor assembly in at least a first dimension and a second mechanical stop configured to limit movement of the sensor assembly in at least a second dimension. A sensor housing may be disposed about the sensor mount and may have a window formed therein to provide fluid communication between the sensor assembly and an environment outside the hypotube.

European Patent Application EP 1658808 A1 discloses a guidewire having pressure sensing capabilities for measuring pressure of a liquid in a vessel comprising a flexible elongate member and having proximal and distal extremities, and having an outside diameter of 0.018" or less. The distal extremity of said flexible elongate member is adapted to be disposed in the liquid in said vessel. A housing is carried by the flexible elongate member and has a diameter substantially the same as the diameter of the flexible elongate member. The housing has a space therein with a pressure sensor mounted in the housing space in the housing. The pressure sensor has a diaphragm formed in a crystal of semiconductor material, that is sensitive to changes in pressure of the liquid in the vessel. The diaphragm is rectangular in shape and is mounted in a well, and is bordered by a rim surrounding the well. A backing plate is formed of an insulating material bonded to the crystal and serves to reinforce the rim of the crystal. The backing plate has a cavity therein underlying the diaphragm and is in substantial registration with the diaphragm. The cavity serves as a pressure reference.

### BRIEF SUMMARY

The invention is defined in independent claim 1. Further aspects are defined in the dependent claims.

The disclosure relates to a catheter, such as a pressure measurement catheter, including an elongate shaft having a proximal end optionally coupled to a handle or luer fitting and a distal end having a distal opening. The elongate shaft further includes a proximal portion, an intermediate portion, and a distal portion having a distal tip. In the proximal portion of the elongated shaft, a shaft wall may define two separate lumens: a guide wire lumen and a second or pressure sensor wire lumen, extending parallel to each other or side-by-side along the proximal portion. The distal portion of the elongate shaft is configured to receive a guidewire in a distal portion of guidewire lumen thereof. The pressure sensing wire may extend to the distal portion of the elongate shaft to be coupled to a pressure sensor mounted on the distal tip for measuring a pressure of a fluid within lumen of vessel. The pressure sensor wire is disposed within a pocket formed adjacent to the pressure sensor thereby minimizing the profile of the catheter.

The disclosure also relates to a catheter, such as a measurement catheter, including an elongate shaft having a proximal end optionally coupled to a handle or luer fitting and a distal end having a distal opening. The elongate shaft further includes a proximal portion, an intermediate portion, and a distal portion having a distal tip. In the proximal portion of elongated shaft, shaft wall may define two separate lumens: a guide wire lumen and a second or pressure sensor wire lumen, extending parallel to each other or side-by-side along the proximal portion. The distal portion of the elongate shaft is configured to receive a guidewire in a distal portion of the guidewire lumen thereof. The pressure sensing wire lumen may extend to the distal portion of the elongate shaft to be coupled to a pressure sensor mounted on the distal tip for measuring a pressure of a fluid within lumen of vessel. A flexible interconnect has one end coupled to the pressure sensor and another end coupled to the pressure sensor wire in order to electrically couple the pressure sensor with the pressure sensor wire. Flexible interconnect not only reduces the profile of the catheter, but also helps to isolate the pressure sensor from the bending stresses applied to the catheter by allowing the pressure sensor and the pressure sensor wire to move independently from one another.

The disclosure also relates to a catheter, such as a measurement catheter, including an elongate shaft having a proximal end optionally coupled to a handle or luer fitting and a distal end having a distal opening. The elongate shaft further includes a proximal portion, an intermediate portion, and a distal portion having a distal tip. In the proximal portion of elongated shaft, shaft wall may define two separate lumens: a guide wire lumen and a second or pressure sensor wire lumen, extending parallel to each other or side-by-side along the proximal portion. The distal portion of the elongate shaft is configured to receive a guidewire in a distal portion of the guidewire lumen thereof. The pressure sensing wire lumen may extend to the distal portion of the elongate shaft to be coupled to a pressure sensor mounted on the distal tip for measuring a pressure of a fluid within lumen of vessel. The pressure sensor and the pressure sensor wire are spaced apart by a gap. The shaft wall is metalized to electrically couple the pressure sensor with the pressure sensor wire. The gap not only reduces the profile of the catheter, but also helps to isolate the pressure sensor from the bending stresses applied to the catheter by allowing the pressure sensor and the pressure sensor wire to move independently from one another.

### BRIEF DESCRIPTION OF DRAWINGS

The foregoing and other features and advantages of the disclosure will be apparent from the following description of embodiments hereof as illustrated in the accompanying drawings. The accompanying drawings, which are incorporated herein and form a part of the specification, further serve to explain the principles of the disclosure and to enable a person skilled in the pertinent art to make and use the disclosure. The drawings are not to scale.
FIG. 1 is a broken view of a system for measuring FFR with a distal portion thereof shown within a vessel including a lesion, the system including a measurement catheter including a pressure sensor and a guidewire, in accordance with an embodiment hereof.
FIG. 2 is a broken view of the catheter of FIG. 1 in partial longitudinal cross-section.
FIG. 3 is a cross-sectional view of the catheter taken along line 3-3 of FIG. 2.
FIG. 4 is a longitudinal cross-sectional view of the distal portion of the catheter of FIG. 1.
FIG. 5A is a longitudinal cross-sectional view of one example of the distal portion of the catheter of FIG. 1.
FIG. 5B is a longitudinal cross-sectional view of another example of the distal portion of the catheter of FIG. 1.
FIG. 6A is a longitudinal cross-sectional view with an interposer shown in the distal portion of the catheter of FIG. 1.
FIG. 6B is a top view of the distal portion of the catheter of FIG. 6A.
FIG. 7 is a longitudinal cross-sectional view of one example of the distal portion of the catheter of FIG. 1.
FIG. 8 is a longitudinal cross-sectional view of an optional embodiment of of the distal portion of the catheter of FIG. 1.

### DETAILED DESCRIPTION

Specific embodiments of the present disclosure are now described with reference to the figures, wherein like reference numbers indicate identical or functionally similar elements. While the disclosure refers to illustrative embodiments for particular applications, it should be understood that the disclosure is not limited thereto. Modifications can be made to the embodiments described herein without departing from the scope of the present disclosure. Those skilled in the art with access to this disclosure will recognize additional modifications, applications, and embodiments within the scope of this disclosure and additional fields in which the disclosed examples could be applied. Therefore, the following detailed description is not meant to be limiting. Further, it is understood that the systems and methods described below can be implemented in many different embodiments of hardware. Any actual hardware described is not meant to be limiting. The operation and behavior of the systems and methods presented are described with the understanding that modifications and variations of the embodiments are possible given the level of detail presented.

References to "one embodiment," "an embodiment," "in certain embodiments," etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described.

Specific embodiments of the present disclosure are now described with reference to the figures, wherein like reference numbers indicate identical or functionally similar elements. The terms "distal" and "proximal" are used in the following description with respect to a position or direction relative to the treating clinician. "Distal" and "distally" are positions distant from or in a direction away from the clinician. "Proximal" and "proximally" are positions near or in a direction toward the clinician.

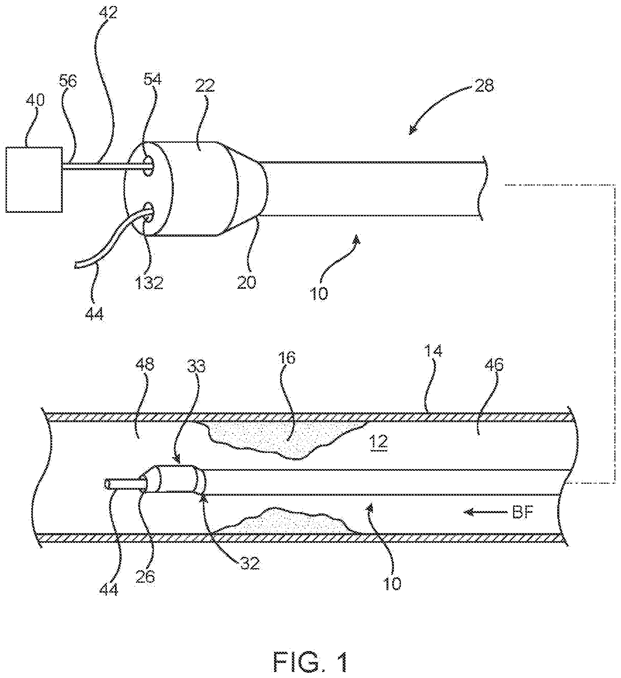

With reference to FIG. 1, a pressure measurement catheter 10 is shown with a proximal portion thereof outside of a patient and a distal portion thereof positioned *in situ* within a lumen 12 of a patient vessel 14 having a stenosis or lesion 16. In an embodiment hereof, the vessel 14 is a blood vessel such as but not limited to a coronary artery. Lesion 16 is generally representative of any blockage or other structural arrangement that results in a restriction to the flow of fluid through lumen 12 of vessel 14. Lesion 16 may be a result of plaque buildup, including without limitation plaque components such as fibrous, fibro-lipidic (fibre fatty), necrotic core, calcified (dense calcium), blood, fresh thrombus, and mature thrombus. Generally, the composition of lesion will depend on the type of vessel being evaluated. In that regard, it is understood that embodiments hereof are applicable to various types of blockage or other narrowing of a vessel that results in decreased fluid flow.

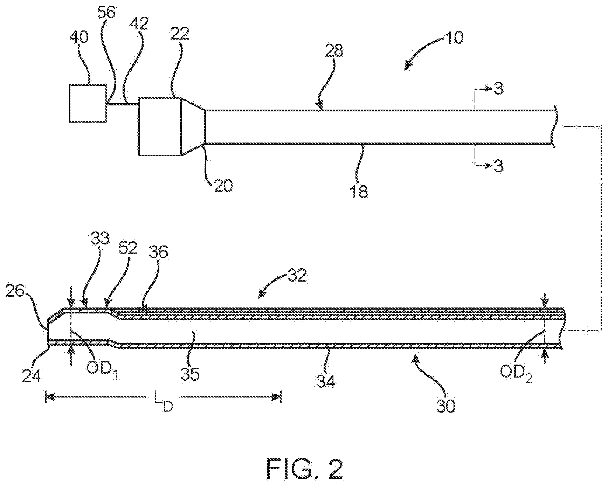

Measurement catheter 10 is shown in FIG. 2 with a distal portion thereof in longitudinal cross-section. Measurement catheter 10 includes an elongate shaft 18 having a proximal end 20 that may be coupled to a handle or luer fitting 22 and a distal end 24 having a distal opening 26. Elongate shaft 18 further includes a proximal portion 28, an intermediate portion 30, and a distal portion 32 having a distal tip 33. Although proximal portion 28, intermediate portion 30, and distal portion 32 of elongate shaft 18 have been described separately, they are described in such a manner for convenience and elongate shaft 18 may be constructed unitarily such that the portions described are part of a unitary shaft. However, different portions of elongate shaft 18 may also be constructed separately and joined together.

In embodiments hereof, elongate shaft 18 or component and/or segments thereof may be formed of polymeric materials, non-exhaustive examples of which include polyethylene terephthalate (PET), polypropylene, polyethylene, polyether block amide copolymer (PEBA), polyamide, fluoropolymers, and/or combinations thereof, either laminated, blended or co-extruded. Optionally, the catheter shaft or some portion thereof may be formed as a composite having a reinforcement material incorporated within a polymeric body in order to enhance strength and/or flexibility. Suitable reinforcement layers include braiding, wire mesh layers, embedded axial wires, embedded helical or circumferential wires, and the like. In one embodiment, for example, at least a proximal portion of elongate shaft 18 may be formed from a reinforced polymeric tube. In other embodiments of an elongate tubular shaft or component in accordance herewith, a proximal segment thereof may be a hypotube of a medical grade stainless steel with outer and inner tubes of a distal segment thereof being formed from any of the polymeric materials listed above.

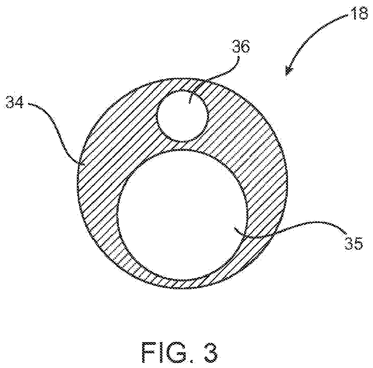

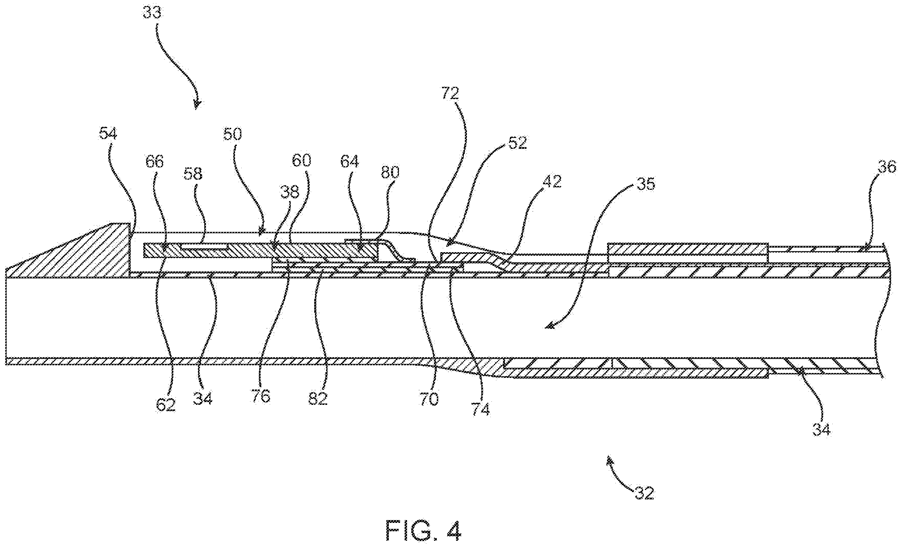

As shown in FIGS. 2-3, elongate shaft 18 has a shaft wall 34 defining a guide wire lumen 35 extending therethrough. Guide wire lumen 35 extends through proximal portion 28, intermediate portion 30, and distal portion 32. However, instead of the over-the-wire configuration shown in FIGS. 1-3, catheter 10 may have a rapid exchange configuration wherein guide wire lumen 35 extends through distal portion 32 and intermediate portion 30, and the guidewire exits shaft 18 through a rapid exchange port (not shown) in proximal portion 28, as would be understood by those skilled in the art. In one embodiment, with reference to the cross-sectional view of FIG. 3 (taken along line 3-3 of FIG. 2), in proximal portion 28 of elongated shaft 18, shaft wall 34 defines two separate lumens, guide wire lumen 35 and a second or pressure sensor wire lumen 36, extending parallel to each other or side-by-side along proximal portion 28. Communication wires 42 are omitted in FIG. 3 for clarity. Although depicted as circular in cross-section, one or more lumen(s) of elongated shaft 18 may have any suitable cross-section including for example circular, elliptical, rectangular or crescent-shaped. As explained in more detail below, pressure sensing wire lumen 36 may extend to distal portion 32 of elongate shaft 18 to be coupled to a pressure sensor 38, as shown in FIGS. 4-5. In one embodiment, pressure sensor wire lumen 36 may be eliminated wherein a signal from pressure sensor 38 is sent to a computing device 40 other than via a wire 42 in a dedicated pressure sensor wire lumen 36, such as, but not limited to, wireless transmission or integration of wire 42 into the wall of elongate shaft 18. In other embodiments of an elongate shaft or tubular component in accordance herewith, pressure sensor wire lumen 36 may be eliminated wherein the shaft or a portion thereof may be formed by a tubular polymeric inner liner overlaid with a power lead layer and a polymeric outer jacket. In such an embodiment, the power leads for the respective pressure sensor of the inner shaft may be wrapped around the respective shaft for all or at least a portion of the shaft and secured in position by the polymeric outer jacket so as to be embedded within the shaft. In another such embodiment, the power lead for the respective pressure sensor of the inner shaft may be straight for a section or for the entire length of the shaft, and secured in position against the inner liner by the polymeric outer jacket so as to be embedded within the shaft.

Distal portion 32 of elongate shaft 18 is configured to receive a guidewire 44 in a distal portion of guidewire lumen 35 thereof. Further, as shown in FIGS. 1, distal portion 32 is sized to extend from a proximal side 46 of lesion 16, through lesion 16, and to a distal side 48 of lesion 16 such that distal tip 33 is disposed on distal side 48 of lesion 16. Accordingly, in an embodiment, distal portion 32 has a length L_{D} in the range of 25-300 mm. However, length L_{D} may be any length suitable such that distal portion 32 may extend from proximal side 46 to distal side 48. Further, because distal portion 32 is configured to extend through lesion 16, the cross-sectional dimension or profile of distal portion 32 is minimized such as to minimize the disruption of blood flow through lesion 16 in order to obtain an accurate FFR measurement.

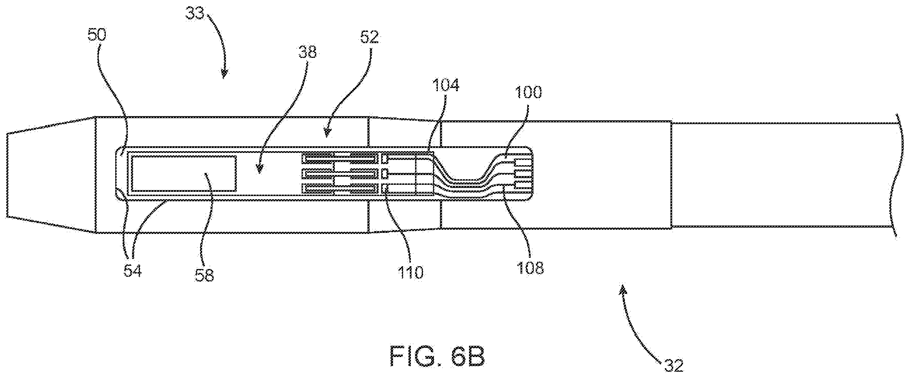

Distal tip 33 is disposed on distal portion 32 of elongate shaft 18. In an optional embodiment (not shown), distal tip 33 is disposed on intermediate portion 30 of elongate shaft 18 and is located proximally of distal portion 32. Distal tip 33 includes pressure sensor 38 for measuring a pressure of a fluid within lumen 12 of vessel 14, as shown in FIG. 4. In the embodiment shown in FIG. 4, pressure sensor 38 is disposed in a pocket 50 (See also FIG. 6B) of a thickened portion 52 of distal tip 33. As shown in FIG. 4, pocket 50 may be defined by at least one substantially vertical sidewall 54 and substantially horizontal shaft wall 34. In another embodiment, pocket 50 has at least one sidewall with a curvilinear shape. Pressure sensor 38 may be a piezo-resistive pressure sensor, a piezo-electric pressure sensor, a capacitive pressure sensor, an electromagnetic pressure sensor, an optical pressure sensor, and/or combinations thereof. In one non-limiting example pressure sensor 38 is a micro electromechanical sensor (MEMS) based pressure die measuring about 240 microns by 70 microns by 1100 microns in size. However, other sized pressure sensors may be used. As shown in FIG. 2, thickened portion 52 needs to accommodate pressure sensor 38. Accordingly, thickened portion 52 of elongate shaft 18 causes tip portion 33 to have an outer diameter OD₁ (shown in Fig 2) which is larger than the outer diameter OD₂ of distal portion 32 of elongate shaft 18. However, depending on the size of pressure sensor 38, the outer diameters OD₁ and OD₂ of the elongate shaft 18 could have substantially the same diameter. In one embodiment, outer diameter OD₁ of tip portion 33 is in the range of 0.024 inch - 0.040 inch in order to accommodate pressure sensor 38. However, outer diameter OD₁ may vary depending on the size of pressure sensor 38, thickness of elongate shaft 18, and other factors used to determine the diameter or profile of shafts. In an optional embodiment, a cover (not shown) could extend substantially over pocket 50 to protect pressure sensor 38 from contacting the vessel wall while still allowing blood to surround pressure sensor 38.

Pocket 50 is in communication with pressure sensor wire lumen 36 such that any communication wire(s) 42 from pressure sensor 38 may extend from pocket 50 proximally through pressure sensor wire lumen 36, through a corresponding lumen in luer fitting 22 exiting through proximal port 54 to a computing device 40 coupled to proximal end 56 of communication wire 42. Proximal end 56 of communication wire 42 may be coupled to computing device 40 via various communication pathways, including but not limited to one or more physical connections including electrical, optical, and/or fluid connections, a wireless connection, and/or combinations thereof. Accordingly, it is understood that additional components (e.g., cables, connectors, antennas, routers, switches, etc.) not illustrated in FIG. 1 may be included to facilitate communication between the proximal end 56 of communication wire 42 and computing device 40. In an optional embodiment, computing device 40 is incorporated into catheter 10 or for example, in proximal portion 28.

FIG. 4 is a longitudinal cross-sectional view of distal shaft portion 32 including distal tip 33. Therein, sensor 38 has a first outwardly facing surface 60, a second inwardly facing surface 62, a first distal end 64 and a second proximal end 66. A diaphragm 58 of sensor 38 is disposed on first surface 60. Communication wires 42 (for example, 0.0025 inch coated copper wire in a tri-filar configuration) extending from lumen 36 are coupled to an electrical interface, such as an interposer 70 which has first and second surfaces 72, 74. In this embodiment, communication wires form an "S-shape", such that one end of the communication wires 42 is raised up to the elevated level of first surface 72 of interposer 70. Second sensor surface 62 is coupled to first surface 72 of interposer 70 (for example, by an adhesive 76), thereby disposing interposer between shaft wall 34 of elongate shaft 18 and sensor 38.

Sensor wires 80 (for example, 0.001 inch thick gold wires) have a first end coupled to first surface 72 of interposer 70 and a second end coupled to electrical pads or metallization on first surface 60 of sensor 38. Similarly to the communication wires, sensor wires may also make an S-shape, such that one end of the sensor wires 80 is raised up to the elevated level of first surface 60 of sensor 38. Interposer 70 has second surface 74 coupled to shaft wall 34 of elongate shaft 18. In one embodiment, interposer 70 is coupled to shaft wall 34 by an adhesive 82 having a thickness of about 25 microns. Sensor 38 may be elevated above shaft wall 34 by the thickness of interposer 70 and to some degree by the thickness of the adhesive layers 76 and 82.

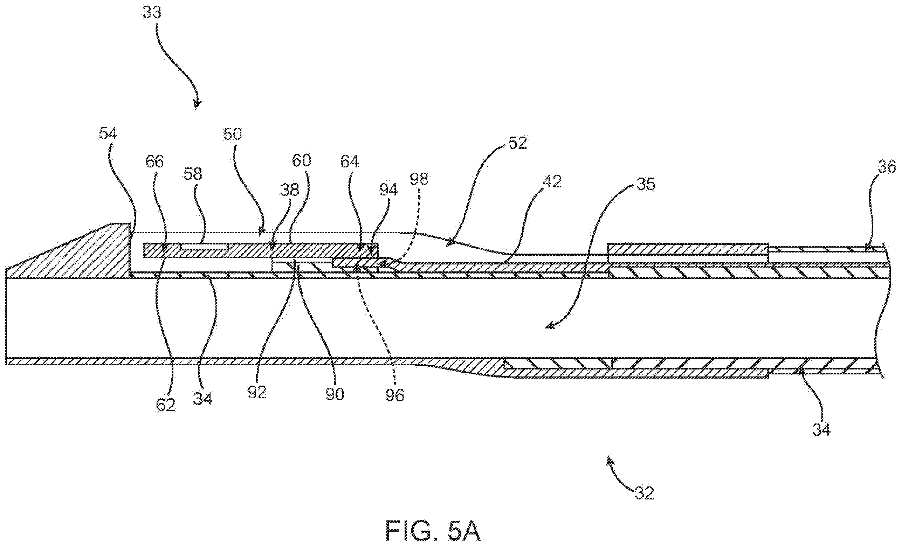

FIG. 5A is a longitudinal cross-sectional view of another example of distal shaft portion 32 including distal tip 33. In FIG. 5A, sensor 38 is elevated above shaft wall 34 by a step 90 extending from shaft wall 34. Sensor 38 is coupled to step 90 by, for example, an adhesive layer 92. Sensor 38 may be elevated above shaft wall 34 by a distance of about 40-50 microns. In another example, the distance between the sensor 38 and shaft wall 34 is about 25-60 microns. Sensor 38 can be coupled to step 90 at any point along the length of sensor 38. As shown in FIG. 5A, sensor 38 is coupled to step 90 at a location that is adjacent first end 64 of sensor 38. Placement of sensor 38 at this location creates an overhang 94, such that first end 64 of sensor 38 is spaced apart from shaft wall 34 thereby forming a pocket 96 under first end 64 of sensor 38. Thus, pocket 96 is defined by step 90, overhang 94 and shaft wall 34. In one example, pocket 96 could be further defined by side walls (not shown) extending from shaft wall 34 on either side of pocket 96, the side walls further extending between step 90 and overhang 94. In an optional example, step 90 extends substantially along the entire second surface 62 (except for overhang 94) of sensor 38 such that second end 62 of sensor is not suspended above shaft wall 34.

In the example of FIG. 5A, pocket 96 has an opening 98 for receiving communication wire 42 (or any type of electrical coupling such as wiring or an interposer) whereby communication wire 42 is coupled to second surface 62 of sensor 38. In this case, sensor 38 could be configured (such as by the flip chip or controlled collapse chip connection method) to have electrical pads, solder bumps or other metallization on second surface 62 instead of first surface 60, in order to provide an electrical coupling between sensor 38 and communication wire 42. Although, communication wire 42 is shown, other wires such as sensor wire 80 are also receivable within pocket 96. Thus, by directly coupling communication wire 42 to second surface 62 of sensor 38 within pocket 96, the example of FIG. 5A (as compared with FIG. 4) has eliminated interposer 70, adhesive layers 76 and 82, and any additional wiring, such as sensor wire 80. By eliminating components needed to create an electrical coupling, the profile of thickened portion 52 is reduced or minimized.

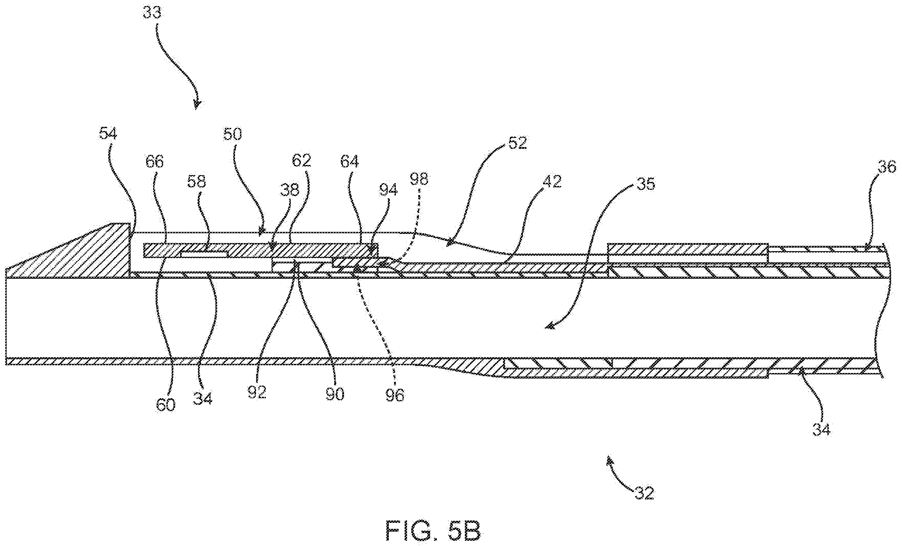

FIG. 5B is a longitudinal cross-sectional view of another example of distal shaft portion 32 including distal tip 33. In this example, sensor 38 is similar to the sensor of FIG. 4 with the electrical pads or other metallization, and diaphragm 58 both disposed on first surface 60 of sensor 38. However, in the example of FIG. 5B, sensor 38 is "flipped" or mounted upside down onto step 90. In this configuration, communication wire 42 is receivable within pocket 96 and coupled to first surface 60 of sensor 38.

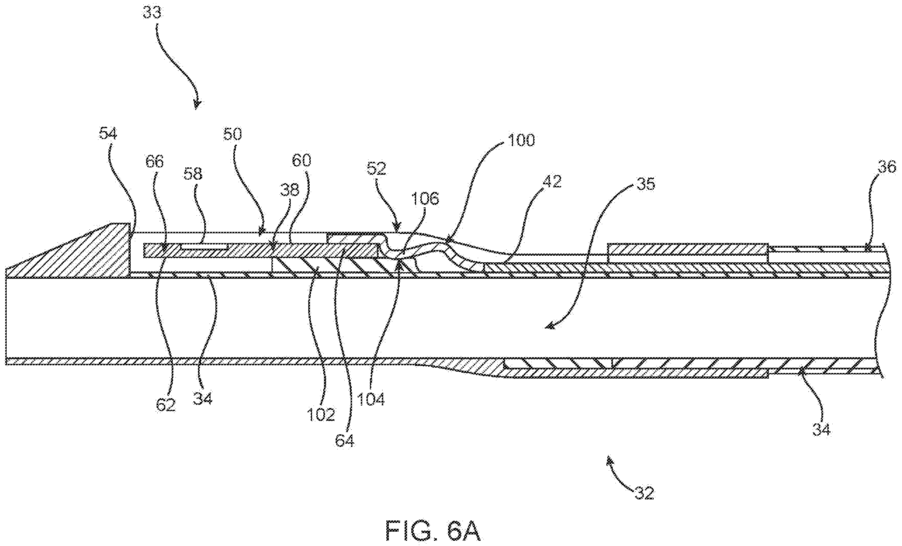

FIG. 6A is a longitudinal cross-sectional view of another example of distal shaft portion 32 including distal tip 33. In addition to reducing the profile of distal tip 33 to a minimum, isolating sensor 38 from any stress or strain is important because physical or mechanical distortion of sensor 38 can result in an incorrect pressure reading. One source of stress or strain applied to sensor 38 could be from the movement of electrical leads, pressure sensor wire 80, or communication wire 42 during operation of catheter 10. To avoid stress and strain from such wiring, one option is to couple one end of a flexible interconnect 100 to communication wire 42 and to couple the other end of flexible interconnect 100 to first surface 60 of sensor 38, as shown in FIG. 6A. Flexible interconnect 100 can be manufactured with cross-sectional profiles as low as 25 microns further reducing the profile of distal tip 33. In one example, sensor 38 is mounted and positioned on one portion of step 102 (which extends from shaft wall 34) in such a way as to form a ledge 104 having a top surface 106. Flexible interconnect 100 can lie against ledge 104 as well as move or slidably curve across top surface 106 of ledge 104 in response to bending forces. Flexible interconnect 100 has elastic and deformable properties that allow flexible interconnect 100 to move, bend and adjust within distal tip 33. The resilient characteristics of flexible interconnect 100 not only help flexible interconnect 100 to minimize the profile of distal tip 33 but also the stress and strain acting on distal tip 33 are absorbed by flexible interconnect 100, instead of sensor 38, further isolating sensor 38 from these bending forces. In addition, flexible interconnect 100 allows sensor 38 and communication wire 42 to move independently from one another, further alleviating the stress and strain being applied to sensor 38.

FIG. 6B is a top view of the embodiment illustrated in FIG. 6A showing flexible interconnect 100 in a curved and compacted configuration to minimize profile of distal tip 33. As can be seen in FIG. 6B, electrical wiring 108 (disposed on or within flexible interconnect 100) couples communication wire 42 to electrical pads 110 of sensor 38. Not only does flexible interconnect 100 minimize the profile of distal tip 33, flexible interconnect 100 also provides a much more stable coupling than, for example, three separate wires coupling communication wire 42 with sensor 38. In addition, separate wiring would require epoxy or solder at each joint to secure wiring to sensor 38, thereby adding to the profile of distal tip 33.

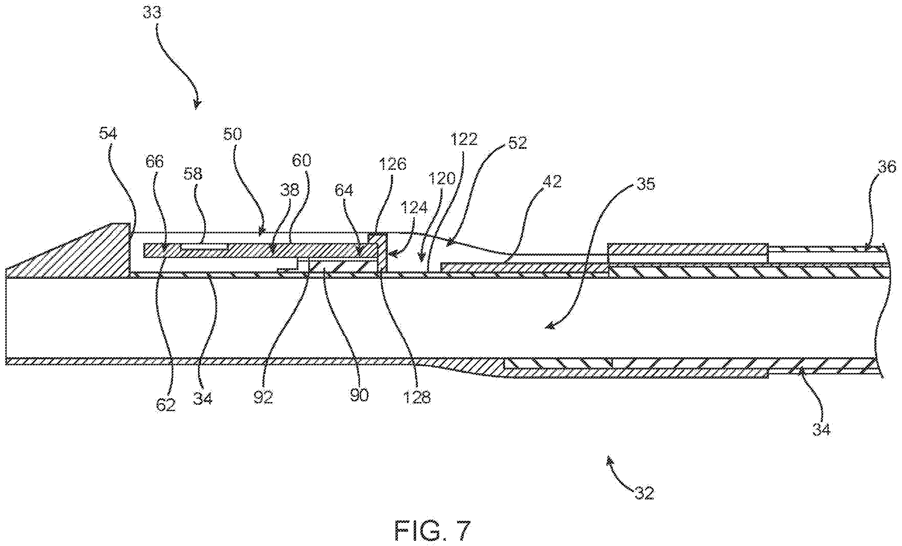

FIG. 7 is a longitudinal cross-sectional view of another example of distal shaft portion 32 including distal tip 33. In the example of FIG. 7, distal tip 33 does not have an interposer or sensor wires. Instead, a gap 120 is provided between sensor 38 and communication wire 42. A top surface 122 of shaft wall 34 which spans the distance of gap 120 is metallized, or electrical leads are etched into top surface 122. As a result, communication wire 42, which is in contact with top surface 122, is electrically coupled to top surface 122 of shaft wall 34. One way to metalize top surface 122 of shaft wall is to mold distal tip 33 with an appropriately doped polymer. Portions of the polymer are exposed to laser direct structuring technology to activate the polymer for selective plating as well as to create patterns for electrical pad configurations. Once the mold is complete, layers of metallization (typically 5-8 microns thick) are placed into the electrical pad patterns thereby electrically coupling sensor 38 with communication wire 42. In an optional example, metallization or electrical leads can be integrated within shaft wall 34 and do not need to extend along top surface 122 of shaft wall 34.

By spanning the distance between sensor 38 and communication wire 42, gap 120 provides a flex region disposed between sensor 38 and communication wire 42. The flex region bends or twists in response to the catheter's movement in the patient's vasculature, which absorbs stress and strain forces that would otherwise be transmitted to sensor 38. The flex region also allows sensor 38 and communication wire 42 to move independently from one another further reducing the amount of stress and strain forces being transmitted to sensor 38. As shown in FIG. 7, a bonding member 124, such as a gold wire bond, is coupled between sensor 38 and top surface 122 of shaft wall 34. More specifically, bonding member 124 has one end 126 coupled to electrical pads 110 of sensor 38, and bonding member 124 has another end 128 coupled to top surface 122 of shaft wall 34. Thus, bonding member 124 provides a bridge to electrically couple sensor 38 to communication wire 42 through top surface 122 of shaft wall 34.

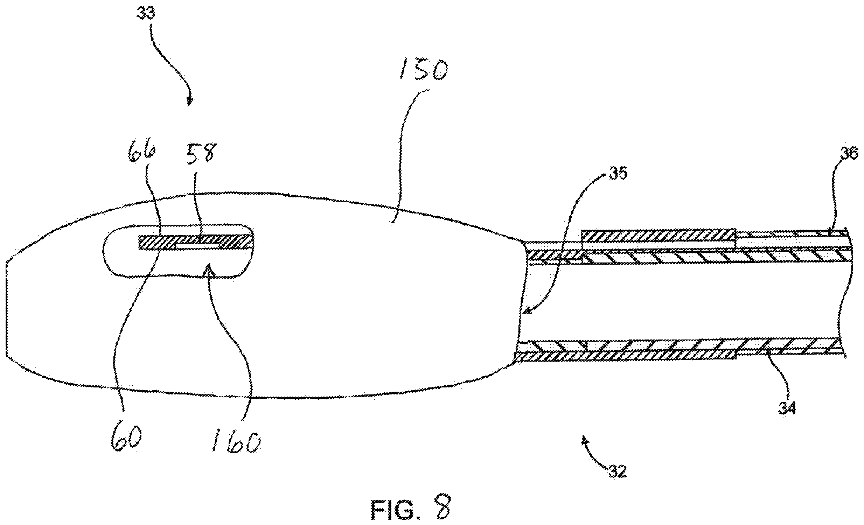

FIG. 8 is a longitudinal cross-sectional view of an optional embodiment of distal shaft portion 32 having a protective covering or cap 150 disposed about and partially or fully enclosing distal tip 33. Although cap 150 could partially or fully enclose an embodiment of any distal tip disclosed herein, cap 150 of FIG. 8 is shown disposed about distal tip 33 of FIG. 5B. With diaphragm 58 facing inward toward surface 34 instead of being exposed directly to fluid in lumen 12, distal tip 33 would need at least one opening 160 in cap 110, preferably, in close proximity to diaphragm 58 to allow ingress of fluid for pressure measurement. In one example, opening 160 would be disposed on the side portion cap 150 (as shown in FIG. 8) such that the opening 160 would be close enough to sensor 38 and diaphragm 58 to provide fluid communication between sensor 38, diaphragm 58 and lumen 12 of patient vessel 14 thereby allowing a pressure measurement by sensor 38. Opening 160 can be positioned anywhere on cap 160 and opening 160 can be of any shape or size depending on the desired amount of fluid communication between patient vessel 14 and sensor 38.

A method of measuring FFR using measurement catheter 10 will now be described with reference to FIG 1. As would be understood by those skilled in the art, when measuring FFR a guide catheter (not shown) may be advanced through the vasculature such that the guide catheter is disposed within the aorta with a distal end thereof disposed within the aorta at an ostium of the aorta adjacent the branch vessel 14 within which lesion 16 is located. As shown in FIG. 1, guidewire 44 can be advanced intraluminally through the guide catheter, into vessel 14 within lumen 12 to the site of lesion 16. In the embodiment shown, guidewire 44 is advanced from proximal side 46 of lesion 16 to distal side 48 of lesion 16, which is also consistent with the direction of the blood flow BF, as indicated by the arrow BF in FIG. 1. In an embodiment, vessel 14 is a coronary artery, but vessel 14 may be other vessels in which it may be desirable to measure pressure, and in particular, to measure FFR.

Thereafter, as shown in FIG. 1, measurement catheter 10 can be tracked or advanced over indwelling guidewire 44 to the target site such that distal end 32 of elongate shaft 18 is positioned distal of lesion 48. As can be seen in FIG. 1, distal tip 33 including pressure sensor 33 can be disposed distally of lesion 16 such that elongate shaft 18 is disposed through lesion 16.

With measurement catheter 10 in place, pressure sensor 33 measures the pressure of blood distal of the lesion within lumen 12. Accordingly, the pressure measured by pressure sensor 33 is the distal pressure measurement, or P_{d}, used in calculating FFR. In one embodiment, adenosine is administered either intracoronary at the site, bolus, or intravenously by continuous infusion for providing an accurate distal pressure measurement (P_{d}) for an FFR value. A proximal pressure measurement Pₐ, which is taken in the aorta by an external AO pressure transducer associated with the guide catheter, and a simultaneous pressure measurement P_{d} taken with pressure sensor 33 of measurement catheter 10 are then obtained to provide the FFR value, *i.e*., P_{d}/Pₐ, for the lesion. The proximal pressure measurement Pₐ and distal pressure measurement P_{d} can be communicated to computing device 40. Computing device 40, shown schematically in FIGS. 1 and 2, may include such components as a CPU, a display device, an amplification and filtering device, an analog-to-digital converter, and various other components. Computing device 40 may receive the proximal pressure measurement Pₐ and distal pressure measurement P_{d}, and may process them to provide a continuous display of FFR measurement.

When the FFR measurement is completed, measurement catheter 10 may then be completely withdrawn from the patient or repositioned *in vivo* at another lesion and the process repeated. Pressure-sensing catheters in accordance with embodiments hereof may be used for other than providing proximal and distal pressure measurements (Pₐ, P_{d}) for calculating an FFR value. For instance, pressure-sensing catheters in accordance with embodiments hereof may be used to provide an in vivo pressure measurement anywhere along the vasculature, or a particular lesion therein. As well, embodiments hereof may be used to provide in vivo pressure measurements, across a heart valve, venous valve or other valvular location within the body where it may be deemed useful.

The detailed description is merely exemplary in nature and is not intended to limit the invention or the application and uses of the invention. Although the description is in the context of treatment of blood vessels such as the coronary arteries, use is also possible in any other body passageways where it is deemed useful such as but not limited to peripheral arteries, carotid arteries, renal arteries, and/or venous applications. Furthermore, there is no intention to be bound by any expressed or implied theory presented in the preceding technical field, background, brief summary or the detailed description.

While various embodiments according to the present disclosure have been described above, it should be understood that they have been presented by way of illustration and example only, and not limitation. It will be apparent to persons skilled in the relevant art that various changes in form and detail can be made therein without departing from the scope of the disclosure. Thus, the breadth and scope of the present disclosure should not be limited by any of the above-described exemplary embodiments. It will also be understood that each feature of each embodiment discussed herein, and of each reference cited herein, can be used in combination with the features of any other embodiment. The invention is defined in the appended claims.

## Claims

1. A catheter comprising:
an elongate shaft (18) including a proximal portion (28) and a distal portion, the elongate shaft (18) having a shaft wall (34), the shaft wall (34) having an outer and inner surface, the shaft wall (34) inner surface defining a guidewire lumen (35);
a pressure sensor (38) coupled to the shaft wall (34) outer surface at the distal end of the elongate shaft (18), the sensor (38) being coupled to the shaft wall (34) such that one end of the sensor (38) is spaced apart from the shaft wall (34) and forms an overhang (94) to define a pocket (96) between the overhang (94) and the shaft wall (34) outer surface; and
an electrical coupling member (42) for electrically coupling the sensor (38),
**characterized in that**
the electrical coupling member (42) is received in the pocket (96).

2. The catheter of claim 1, wherein the sensor (38) has first and second surfaces and a diaphragm on the first surface, further wherein the electrical coupling member (42) is coupled to the second surface of the sensor (38).

3. The catheter of claim 1, wherein the sensor (38) has first and second surfaces and a diaphragm on the first surface, the sensor (38) is coupled to the shaft wall (34) on the first surface, further wherein the electrical coupling member (42) is coupled to the first surface of the sensor (38).

4. The catheter of claim 1, wherein the sensor (38) is mounted to a step (90) extending from the shaft wall (34).

5. The catheter of claim 4, wherein the sensor (38) is mounted to the step (90) at a location that is adjacent to one end of the sensor (38) thereby forming the overhang (94).

6. The catheter of claim 5, wherein another end of the sensor (38) is spaced apart from the shaft wall (34).

7. The catheter of claim 4, wherein the pocket (96) is defined by the overhang (94), the step (90) and the shaft wall (34).

8. The catheter of claim 4, wherein the pocket (96) is further defined by side walls extending from shaft wall (34), side walls are disposed on either side of pocket (96) between step (90) and overhang (94).

9. The catheter of any of preceding claims, wherein the electrical coupling member is a communication wire (42) to provide communication between the sensor (38) and a computing device.

10. The catheter of any of claims 1 to 8, wherein the electrical coupling member (42) is an interposer.

## Patentansprüche

1. Katheter, der Folgendes umfasst:
einen länglichen Schaft (18), der einen proximalen Abschnitt (28) und einen distalen Abschnitt aufweist, wobei der längliche Schaft (18) eine Schaftwand (34) aufweist, wobei die Schaftwand (34) eine äußere und innere Oberfläche aufweist, wobei die innere Oberfläche der Schaftwand (34) ein Führungsdrahtlumen (35) definiert;
einen mit der äußeren Oberfläche der Schaftwand (34) verbundenen Drucksensor (38) am distalen Ende des länglichen Schaftes (18), wobei der Sensor (38) mit der Schaftwand (34) derart verbunden ist, dass ein Ende des Sensors (38) von der Schaftwand (34) beabstandet ist und einen Überhang (94) bildet, um eine Tasche (96) zwischen dem Überhang (94) und der äußeren Oberfläche der Schaftwand (34) zu bilden; und
ein elektrisches Kopplungselement (42) zum elektrischen Koppeln des Sensors (38), **dadurch gekennzeichnet, dass** das elektrische Kopplungselement (42) in der Tasche (96) aufgenommen ist.

2. Katheter nach Anspruch 1, wobei der Sensor (38) erste und zweite Oberflächen und eine Membran auf der ersten Oberfläche aufweist, ferner wobei das elektrische Kopplungselement (42) mit der zweiten Oberfläche des Sensors (38) gekoppelt ist.

3. Katheter nach Anspruch 1, wobei der Sensor (38) erste und zweite Oberflächen und eine Membran auf der ersten Oberfläche aufweist, wobei der Sensor (38) mit der Schaftwand (34) auf der ersten Oberfläche verbunden ist, ferner wobei das elektrische Kopplungselement (42) mit der ersten Oberfläche des Sensors (38) verbunden ist.

4. Katheter nach Anspruch 1, wobei der Sensor (38) an einem Absatz (90) angebracht ist, der sich von der Schaftwand (34) erstreckt.

5. Katheter nach Anspruch 4, wobei der Sensor (38) an dem Absatz (90) an einer Position angebracht ist, die benachbart zu einem Ende des Sensors (38) ist, wodurch der Überhang (94) gebildet wird.

6. Katheter nach Anspruch 5, wobei ein anderes Ende des Sensors (38) von der Schaftwand (34) beabstandet ist.

7. Katheter nach Anspruch 4, wobei die Tasche (96) durch den Überhang (94), den Absatz (90) und die Schaftwand (34) definiert ist.

8. Katheter nach Anspruch 4, wobei die Tasche (96) ferner durch Seitenwände, die sich von der Schaftwand (34) erstrecken, definiert ist, wobei Seitenwände auf jeder Seite der Tasche (96) zwischen Absatz (90) und Überhang (94) angeordnet sind.

9. Katheter nach einem der vorstehenden Ansprüche, wobei das elektrische Kopplungselement ein Kommunikationsdraht (42) ist, um eine Kommunikation zwischen dem Sensor (38) und einer Computervorrichtung bereitzustellen.

10. Katheter nach einem der Ansprüche 1 bis 8, wobei das elektrische Kopplungselement (42) ein Interposer ist.

## Revendications

1. Cathéter comprenant :
une tige allongée (18) incluant une partie proximale (28) et une partie distale, la tige allongée (18) ayant une paroi de tige (34), la paroi de tige (34) ayant une surface externe et interne, la surface interne de la paroi de tige (34) définissant une lumière de fil-guide (35) ;
un capteur de pression (38) couplé à la surface externe de la paroi de tige (34) au niveau de l'extrémité distale de la tige allongée (18), le capteur (38) étant couplé à la paroi de tige (34) de telle sorte qu'une extrémité du capteur (38) est espacée de la paroi de tige (34) et forme un surplomb (94) pour définir une poche (96) entre le surplomb (94) et la surface externe de la paroi de tige (34) ; et
un élément de couplage électrique (42) pour coupler électriquement le capteur (38), **caractérisé en ce que** l'élément de couplage électrique (42) est reçu dans la poche (96).

2. Cathéter selon la revendication 1, dans lequel le capteur (38) a des première et deuxième surfaces et un diaphragme sur la première surface, dans lequel en outre l'élément de couplage électrique (42) est couplé à la deuxième surface du capteur (38).

3. Cathéter selon la revendication 1, dans lequel le capteur (38) a des première et deuxième surfaces et un diaphragme sur la première surface, le capteur (38) est couplé à la paroi de tige (34) sur la première surface, dans lequel en outre l'élément de couplage électrique (42) est couplé à la première surface du capteur (38).

4. Cathéter selon la revendication 1, dans lequel le capteur (38) est monté sur un étagement (90) s'étendant à partir de la paroi de tige (34).

5. Cathéter selon la revendication 4, dans lequel le capteur (38) est monté sur l'étagement (90) à un emplacement qui est adjacent à une extrémité du capteur (38) en formant de ce fait le surplomb (94).

6. Cathéter selon la revendication 5, dans lequel une autre extrémité du capteur (38) est espacée de la paroi de tige (34).

7. Cathéter selon la revendication 4, dans lequel la poche (96) est définie par le surplomb (94), l'étagement (90) et la paroi de tige (34).

8. Cathéter selon la revendication 4, dans lequel la poche (96) est en outre définie par des parois latérales s'étendant à partir de la paroi de tige (34), les parois latérales sont disposées de chaque côté de la poche (96) entre l'étagement (90) et le surplomb (94).

9. Cathéter selon l'une quelconque des revendications précédentes, dans lequel l'élément de couplage électrique est un fil de communication (42) pour fournir une communication entre le capteur (38) et un dispositif informatique.

10. Cathéter selon l'une quelconque des revendications 1 à 8, dans lequel l'élément de couplage électrique (42) est un interposeur.
